(19) Europäisches Patentamt
European Patent Office
Office européen des brevets

(11) Veröffentlichungsnummer: **0 016 433**
**B1**

(12) # EUROPÄISCHE PATENTSCHRIFT

(45) Veröffentlichungstag der Patentschrift: **28.07.82**

(51) Int. Cl.³: **C 07 C 29/66,** C 07 C 31/36

(21) Anmeldenummer: **80101341.8**

(22) Anmeldetag: **14.03.80**

(54) **Verfahren zur kontinuierlichen Herstellung von Propylenchlorhydrinen.**

(30) Priorität: **19.03.79 DE 2910675**

(43) Veröffentlichungstag der Anmeldung: **01.10.80 Patentblatt 80/20**

(45) Bekanntmachung des Hinweises auf die Patenterteilung: **28.07.82 Patentblatt 82/30**

(84) Benannte Vertragsstaaten: **BE DE FR GB NL**

(56) Entgegenhaltungen:
BE-A-410 831
DE-A-2 119 214
DE-B-1 063 140
DE-B-2 022 819
FR-A-2 025 850
FR-A-2 076 365
GB-A-489 576
US-A-1 875 309
US-A-3 093 690

„Journal of the Council for Scientific and Industrial Research", Bd. 17, Nr. 4, November 1944, S. 213-221 Melbourne (Australien), K.E. MURRAY: „Ethylene Chlorhydrin: A Laboratory Investigation of a Continuous Process for its Production on a Commercial Scale".

(73) Patentinhaber: **BASF Aktiengesellschaft, Carl-Bosch-Strasse 38, D-6700 Ludwigshafen (DE)**

(72) Erfinder: **Koehler, Waldemar, Dr., Max-Slevogt-Strasse 28, D-6710 Frankenthal (DE)**
Erfinder: **Langensiepen, Hans-Werner, Dr. Dipl.-Ing., Limburgstrasse 87, D-6702 Bad Duerkheim 1 (DE)**
Erfinder: **Volkamer, Klaus, Dr., Heidelberger Ring 21, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 016 433 B1

ACTORUM AG

# Verfahren zur kontinuierlichen Herstellung von Propylenchlorhydrinen

Die vorliegende Erfindung betrifft ein verbessertes Verfahren zur kontinuierlichen Herstellung von Propylenchlorhydrinen durch Umsetzung von Chlor und Propylen in wässriger Lösung.

Dieses Verfahren, welches nach folgender Reaktionsgleichung

$$CH_3-CH=CH_2 + Cl_2$$

$$+ H_2O \rightarrow \sim 80\%\ CH_3-\underset{OH}{\underset{|}{C}}H-\underset{Cl}{\underset{|}{C}}H_2$$

α-Propylenchlorhydrin

$$+ \sim 20\%\ CH_3-\underset{Cl}{\underset{|}{C}}H-\underset{OH}{\underset{|}{C}}H_2 + HCl$$

β-Propylenchlorhydrin

verläuft ist, sieht man von der erfindungsgemässen Verbesserung ab, in zahlreichen Ausführungsformen allgemein bekannt, u.a. z.B. aus der US-PS Nr. 2902519, der DE-PS Nr. 1063140, der DE-PS Nr. 1028109 und der DE-AS Nr. 2022819. Allen diesen Verfahrensweisen ist gemeinsam, dass man Chlor und Propylen zusammen mit einem grossen Überschuss an Wasser kontinuierlich bei Normaldruck oder leicht erhöhtem Druck in einem Reaktor oder mehreren hintereinander geschalteten Reaktoren miteinander umsetzt und einen Teil der hierbei erhaltenen salzsauren Propylenchlorhydrinlösung wieder in den Reaktor, bzw. die Reaktoren zurückführt.

Da diese Lösungen kaum noch Chlor enthalten und da das meist in etwas überschüssiger Menge eingesetzte Propylen unschwer aus den Lösungen abgetrieben werden kann, wäre die Teilrückführung an sich überflüssig, wenn sich das Chlor nicht weit besser in wässriger Salzsäure als in Wasser allein lösen würde. Wollte man die Rückführung daher weglassen, so müsste man, um in homogenflüssiger Phase arbeiten zu können, erheblich grössere Wassermengen und entsprechend grössere Apparaturen verwenden.

Aus der GB-PS Nr. 489576 war es weiterhin bekannt, ein Gemisch aus Wasser und einem Olefin einerseits sowie Chlor und einem Inertgas andererseits in einen Strom aus kaltem Wasser einzuführen und dort bei Normaldruck zum entsprechenden Chlorhydrin reagieren zu lassen. Dieses Verfahren ist allein deswegen nachteilig, weil es bei relativ tiefen Temperaturen (etwa 5 bis 20° C) ausgeführt werden muss und weil es die Mitverwendung eines Inertgases erfordert. Durch diese Massnahmen wird die Raum/Zeit-Ausbeute am Verfahrensprodukt notwendigerweise soweit herabgesetzt, dass das Verfahren für technische Zwecke nicht in Betracht kommt.

Ferner war aus der US-PS Nr. 1875309 die Herstellung von Alkylenchlorhydrinen bekannt, in der eine gasphasenfreie Lösung aus Wasser und einem Olefin unter einem Druck von mindestens 40 bar, generell jedoch bei 100 bis 200 bar, mit Hypochlorsäure, die ihrerseits aus CaO und $Cl_2$ hergestellt wird, umgesetzt wird. Diese Verfahrensweise ist schon deshalb für die Technik unbrauchbar, weil es die Mitverwendung stöchiometrischer Mengen CaO sowie die Aufbereitung der hierbei entstehenden wertlosen wässrigen $CaCl_2$-Lösungen erfordert.

Beide vorgenannten Verfahren kommen zwar ohne Rückführung des Reaktionsgemisches aus, dies jedoch unter Inkaufnahme der geschilderten weit grösseren Nachteile.

Obwohl die Rückführung nach den bisherigen Kenntnissen für technische Verfahren unerlässlich ist, lag der Erfindung die Aufgabe zugrunde, sie durch wirtschaftlichere Massnahmen zu ersetzen.

Dementsprechend wurde ein Verfahren zur kontinuierlichen Herstellung von Propylenchlorhydrinen durch Umsetzung von Chlor und Propylen in wässriger Lösung bei 20 bis 80° C und erhöhtem Druck gefunden, welches dadurch gekennzeichnet ist, dass man die Ausgangsstoffe Chlor, Propylen und Wasser in gleichgerichteter Strömung unter einem Druck von 15 bis 40 bar umsetzt, ohne einen Teilstrom des Umsetzungsgemisches wieder in die Umsetzungszone zurückzuführen.

Durch die erfindungsgemässe Verbesserung erhöhen sich die Raum/Zeit-Ausbeuten erheblich. Ausserdem wurde im Zuge der Untersuchungen überraschenderweise ein merklicher Rückgang an unerwünschten Nebenprodukten wie hauptsächlich Dichlorpropan festgestellt. Nach den bisherigen Beobachtungen ist die Ursache hierfür in der durch den Wegfall der Rückführung der salzsauren Lösung bedingten verminderten Chloridionenkonzentration zu suchen. Ein weiterer, durch den Wegfall der Rückführung bedingter Vorteil liegt darin, dass ein Destillationsschritt entfällt, der im Falle der Rückführung zur laufenden Abreicherung der Nebenprodukte im Kreislauf erforderlich ist.

Propylen und Chlor werden vorzugsweise im Molverhältnis von (1,0 bis 2,0) : 1 eingesetzt. Die Menge des Wassers beträgt vorzugsweise 10 bis 50 l/kg Chlor. Die Reaktionstemperatur liegt zweckmässigerweise zwischen 30 und 70° C und der Druck zwischen 20 und 30 bar. Man kann die Reaktion isotherm oder vorzugsweise adiabatisch vornehmen, wobei sich im letztgenannten Falle die Temperatur vom Eingang bis zum Ausgang des Reaktors etwa um 40° C erhöht.

Vorzugsweise gibt man die Ausgangsstoffe von unten in feiner Verteilung gasförmig oder flüssig in einen Rohrreaktor, wobei man dafür sorgt, dass keine nennenswerte, die Bildung von Nebenprodukten begünstigende Rückvermischung stattfindet. Weiterhin empfiehlt es sich, die Ausbildung einer Gasphase im Reaktor zu vermeiden, um dadurch der Bildung der Nebenprodukte entgegenzuwirken.

Anstelle eines einzigen Reaktors kann man auch mehrere, vorzugsweise zwei bis vier, Reaktoren hintereinanderschalten. Hierbei empfiehlt es sich,

sowohl die gesamte Menge an Chlor als auch an Propylen durch jeweils getrennte Zugabe zu den einzelnen Reaktoren entsprechend den in ihnen erzielbaren Umsätzen aufzuteilen. Auch bei der mehrstufigen Fahrweise ist keine Rückführung der Reaktionslösung in einen der vorgeschalteten Reaktoren vorgesehen.

Im übrigen kann das erfindungsgemässe Verfahren nach den üblichen Techniken vorgenommen werden; das gleiche gilt auch für die Aufarbeitung der erhaltenen wässrig-salzsauren Lösungen auf die Propylenchlorhydrine oder auf Propylenoxid. Selbstverständlich kann das hierbei anfallende überschüssige Propylen in den Verfahrenskreislauf zurückgeführt werden.

*Beispiel*

Ein Reaktionsrohr von 20 m Länge und 6 mm Innendurchmesser wurde pro Stunde mit 100 l Wasser, 2 kg Propylen und 3 kg Chlor (Molverhältnis Propylen: Chlor = 1,02: 1) beschickt.

Das Rohr stand unter einem Druck von 29 bar an der Eintrittsstelle und 25 bar am Ausgang, und die Reaktionstemperaturen betrugen entsprechend 30 bzw. 70° C. Unter diesen Betriebsbedingungen konnte sich keine für die Reaktion nachteilige Gasphase ausbilden.

Die Reaktionslösung enthielt 4,7 Gew.% Propylenchlorhydrin entsprechend einer Ausbeute von 95%, bezogen auf das eingesetzte Chlor. Daneben fielen noch 5% Nebenprodukte wie hauptsächlich Dichlorpropan an. Aus dem Volumen des Rohrs, dem Durchsatz und der Propylenchlorhydrinausbeute errechnet sich eine Raum/Zeit-Ausbeute von rund 16 $kg/l^{-1}/h^{-1}$.

In einem Vergleichsversuch gemäss der Verfahrensweise der DE-AS Nr. 2022819 wurde der obige Versuch mit dem Unterschied wiederholt, dass der Druck 4 bar betrug und dass ein Teilstrom von 240 l/h Reaktionsgemisch zurückgeführt wurde, wobei ein Reaktionsrohr mit entsprechend grösserem Querschnitt verwendet wurde.

Die Ausbeute an Propylenchlorhydrin betrug in diesem Fall 94%. Bezogen auf die Propylenchlorhydrine und auf das Reaktorvolumen entspricht dies einer Raum/Zeit-Ausbeute von 11 $kg/l^{-1}/h^{-1}$. Die Rückführung machte es im kontinuierlichen Betrieb ausserdem erforderlich, einen Teil der Nebenprodukte laufend destillativ aus dem Kreislauf zu entfernen, um eine Anreicherung dieser Nebenprodukte zu unterbinden.

## Patentanspruch

Verfahren zur kontinuierlichen Herstellung von Propylenchlorhydrin durch Umsetzung von Chlor und Propylen in wässriger Lösung bei 20 bis 80° C und erhöhtem Druck, dadurch gekennzeichnet, dass man die Ausgangsstoffe Chlor, Propylen und Wasser in gleichgerichteter Strömung unter einem Druck von 15 bis 40 bar umsetzt, ohne einen Teilstrom des Umsetzungsgemisches wieder in die Umsetzungszone zurückzuführen.

## Claim

A process for the continuous preparation of propylene chlorhydrin by reacting clorine and propylene in aqueous solution at from 20 to 80° C under superatmospheric pressure, characterized in that the starting materials, chlorine, propylene and water, flowing in co-current, are reacted under a pressure of from 15 to 40 bar without recycling a part-stream of the reaction mixture to the reaction zone.

## Revendication

Procédé pour la préparation en continu de chlorhydrine de propylène par réaction de chlore et de propylène en solution aqueuse à une température de 20 à 80° C et sous une pression élevée, caractérisé en ce qu'on fait réagir les substances de départ, chlore, propylène et eau s'écoulant dans le même sens, sous une pression de 15 à 40 bar, sans recycler dans la zone de réaction un courant partiel du mélange réactionnel.